# EUROPEAN PATENT APPLICATION

(11) **EP 2 857 044 A1**
(43) Date of publication of application: **08.04.2015**
(21) Application number: 13796522.4
(22) Date of filing: 30.05.2013
(51) Int. Cl.: A61K 48/00, A61K 9/14, A61K 35/76, A61K 47/36, A61K 47/48, C12N 15/09

(54) **CARRIER FOR GENE INTRODUCTION, GENE INTRODUCTION AGENT, METHODS FOR PRODUCING SAID CARRIER AND SAID GENE INTRODUCTION AGENT, AND METHOD FOR INTRODUCING GENE INTO CELL**

(30) Priority: 30.05.2012 JP 2012123305
(71) Applicant: LSIP, LLC, Chiyoda-ku Tokyo 100-0005 (JP)
(72) Inventor: HANDA, Hiroshi, Tokyo 152-8550 (JP); AKAMOTO, Satoshi, Tokyo 152-8550 (JP); HATAKEYAMA, Mamoru, Okayama-shi, Okayama 700-0817 (JP)
(74) Representative: Cabinet Plasseraud
(86) International application number: PCT/JP2013/003415
(87) International publication number: WO 2013/179669

(57) **Abstract**

The present invention provides; a novel gene introduction method which enables a gene to be introduced more safely and more freely, particularly a method for introducing a gene into a specified site in the brain safely and freely; a carrier for gene introduction use, which comprises a nano-particle and a substance capable of binding to a vector for gene introduction and has functional groups involved in the induction of phagocytosis by cells, wherein the substance capable of binding to a vector for gene introduction can bind to the surface of the nano-particle through some of the functional groups and another some of the functional groups remain unbound to the substance capable of binding to a vector for gene introduction ; and a gene introduction agent, in which a vector for gene introduction is bound to the substance capable of binding to a vector for gene introduction in the carrier for gene introduction.

## Description

### Technical Field

The present invention relates to a gene introduction agent characterized in that heparin or heparan sulfate is bound to the surface, and a vector for gene introduction is bound through heparin or heparan sulfate, a gene introduction method using the gene introduction agent, and a method for producing the gene introduction agent.

### Background Art

Previously, a gene introduction method for making a target cell, a tissue and a target organ express an objective gene using a virus with an objective gene incorporated therein has been developed. For example, a method for injecting a virus with a gene incorporated therein into a blood vessel, and transferring the virus to a target organ by the blood flow, and a method for directly injecting the virus into a target organ using a syringe or the like have been developed. However, in such methods, there are problems that an injected virus is diffused, the virus concentration at a desired site is reduced, the sufficient gene expression level cannot be obtained, and the gene is expressed at a site which is not a desired site and is likely to cause an unpreferable action.

In addition, there is a method for attaching a nucleic acid or a virus to a magnetic particle coated with a biological molecule, and accumulating the magnetic particle to a predetermined place by the external magnetic field to introduce a gene (Patent Literature 2). And, there is also a method for binding a virus to a fibrous structure bound with heparin, introducing the fibrous structure into a target organ or the like, and thereby, selectively infecting a cell which has contacted with the fibrous structure with a virus to express a gene (Patent Literature 1). In addition, a bead which has become to have an amino group by addition of polylysine (NH2 residue-type bead) has been developed, and the bead has the nerve fiber elongation effect.

However, even currently, it is stated that the technique for introducing a gene more safely and more freely is necessary. Particularly, since a burden on a patient requiring stereotaxy is large, such technique is required, for example, in introduction of a gene into a brain, such as gene therapy for Parkinson's disease.

### Prior Art Literatures

### Patent Literatures

Patent Literature 1: JP-2011-201793 A
Patent Literature 2: International Publication No. 2005/095621
Patent Literature 3: JP-2006-88131 A
Patent Literature 4: JP-2008-500049 A
Patent Literature 5: JP-2008-127454 A

### Non-Patent Literatures

Non-Patent Literature 1: PNAS, Vol. 106, No. 1, pp44-49 (2009)
Non-Patent Literature 2: Infect. Immun. 1984, 43(2): 561
Non-Patent Literature 3: Infect. Immun. February 1984 vol. 43 no. 2 561-566
Non-Patent Literature 4: J Biomed Mater Res A. 2005 Mar 15; 72 (4) : 389-98

### Disclosure of Invention

### Problems to be Solved by Invention

An object of the present invention is to provide a new gene introduction method which enables a gene to be introduced more safely and more freely. Particularly, an object of the present invention is to provide a method for introducing a gene into a specified site in an organ safely and freely.

### Means for Solving the Problems

In order to attain the aforementioned object, the present inventors found out that, when heparin is bound to the surface of a nano-particle through an amino group, an adeno-associated virus vector is further attached, and this is injected into a specified site in a brain of a rat, the adeno-associated virus vector migrates to a nerve cell which has contacted with the nano-particle, and thereafter, the nano-particle is removed from a brain by phagocytosis of a macrophage, resulting in completion of the present invention.

Based on this finding, the present invention provides the following (i) to (xiv).
(i) A carrier for gene introduction comprising a nano-particle and a substance capable of binding to a vector for gene introduction, wherein
   the carrier has functional groups involved in the induction of phagocytosis by cells,
   the substance capable of binding to a vector for gene introduction can bind to the surface of the nano-particle through some of the functional groups, and
   another some of the functional groups remain unbound to the substance capable of binding to a vector for gene introduction.
(ii) A gene introduction agent, characterized in that a vector for gene introduction is bound to a substance capable of binding to a vector for gene introduction in the carrier for gene introduction according to (i).
(iii) The gene introduction agent according to (ii), wherein the vector for gene introduction is one or more selected from the group consisting of a sendaivirus vector, a lentivirus vector, a retrovirus vector, an adenovirus vector and an adeno-associated virus vector.
(iv) The gene introduction agent according to (ii) or (iii), wherein the functional group(s) has a positive charge.
(v) The gene introduction agent according to any one of (ii) to (iv), wherein the functional group (s) is an amino group.
(vi) The gene introduction agent according to any one of (ii) to (v), wherein an average particle diameter of the nano-particle is 10 nm to 1000 nm.
(vii) The gene introduction agent according to any one of (ii) to (vi), wherein the substance capable of binding to a vector for gene introduction is heparin and/or heparan sulfate.
(viii) The gene introduction agent according to any one of (ii) to (vii), wherein the nano-particle has magnetism.
(ix) A method for introducing a gene into a cell comprising using a gene introduction agent which comprises a nano-particle, a vector for gene introduction, and a substance capable of binding to a vector for gene introduction, wherein
   the agent has functional groups involved in the induction of phagocytosis by cells,
   the substance capable of binding to a vector for gene introduction can bind to the surface of the nano-particle through some of the functional groups,
   another some of the functional groups remain unbound to the substance capable of binding to a vector for gene introduction, and
   the vector for gene introduction is bound to the substance capable of binding to a vector for gene introduction.
(x) A method for introducing a gene into a cell, comprising a procedure of binding a vector for gene introduction to a substance capable of binding to a vector for gene introduction, in a carrier for gene introduction comprising a nano-particle and the substance capable of binding to a vector for gene introduction, wherein
   the carrier has functional groups involved in the induction of phagocytosis by cells,
   the substance capable of binding to a vector for gene introduction can bind to the surface of the nano-particle through some of the functional groups, and
   another some of the functional groups remain unbound to the substance capable of binding to a vector for gene introduction,
   to prepare a gene introduction agent.
(xi) The gene introduction method according to (ix) or (x), comprising a procedure of guiding the gene introduction agent to a target cell by injection.
(xii) The gene introduction method according to (xi), wherein the nano-particle has magnetism, and the method comprises a procedure of applying the magnetic field to the gene introduction agent to retain it at an injection site.
(xiii) A method for producing a carrier for gene introduction comprising a nano-particle having functional groups involved in the induction of phagocytosis by cells on the surface and a substance capable of binding to a vector for gene introduction, comprising
   a step of binding the substance capable of binding to a vector for gene introduction to the nano-particle through some of the functional groups.
(xiv) A method for producing a gene introduction agent comprising a step of binding a vector for gene introduction to a substance capable of binding to a vector for gene introduction in the carrier for gene introduction obtained by the production method according to (xiii).

Also, the present invention provides the following [1] to [13].
[1] A gene introduction agent for introducing a gene into a target cell, a target tissue, or a target organ, comprising a nano-particle, characterized in that the nano-particle is a nano-particle in which heparin or heparan sulfate is bound to the surface through a bond, and a vector for gene introduction is bound thereto through heparin or heparan sulfate.
[2] The gene introduction agent according to [1], wherein the bond is a chemical bond through an amino group, a thiol group or an active ester group on the nano-particle, or a physical bond.
[3] The gene introduction agent according to [1] or [2], wherein the nano-particle has a size of 10 nm to 1000 nm.
[4] The gene introduction agent according to any one of [1] to [3], wherein the nano-particle is a magnetic particle.
[5] The gene introduction agent according to any one of [1] to [4], wherein the vector for gene introduction is selected from the group consisting of a sendaivirus vector, a lentivirus vector, a retrovirus vector, an adenovirus vector, and an adeno-associated virus vector.
[6] A gene introduction method comprising introducing a gene into a non-human mammal or an isolated target cell using a nano-particle, wherein the nano-particle is a nano-particle characterized in that heparin or heparan sulfate is bound to the surface through a bond, and a vector for gene introduction is bound thereto through heparin or heparan sulfate.
[7] The gene introduction method according to [6], wherein the bond is a chemical bond through an amino group, a thiol group, or an active ester group, or a physical bond.
[8] The gene introduction method according to [6] or [7], wherein the nano-particle has a size of 10 nm to 1000 nm.
[9] The gene introduction method according to any one of [6] to [8], comprising a step of guiding the nano-particle to a target cell by injection.
[10] The gene introduction method according to any one of [6] to [9], wherein the nano-particle is a magnetic particle, and the method further comprises a step of applying the magnetic field to guide the magnetic particle to the target cell.
[11] The gene introduction method according to any one of [6] to [10], wherein the vector for gene introduction is selected from the group consisting of a sendaivirus vector, a lentivirus vector, a retrovirus vector, an adenovirus vector, and an adeno-associated virus vector.
[12] A method for producing a gene introduction agent comprising a nano-particle, comprising:
   (i) a step of reacting an amino group donating compound or a thiol group donating compound with the nano-particle to bind an amino group or a thiol group to the nano-particle surface,
   (ii) a step of binding heparin or heparan sulfate to the nano-particle with an amino group or a thiol group bound thereto, and
   (iii) a step of binding a vector for gene introduction thereto through heparin or heparan sulfate.
[13] The production method according to [12], wherein the vector for gene introduction is selected from the group consisting of a sendaivirus vector, a lentivirus vector, a retrovirus vector, an adenovirus vector, and an adeno-associated virus vector.

In the present invention, the "functional group involved in the induction of phagocytosis by cells" means a functional group which can be recognized by a macrophagic cell to induce phagocytosis of the cell. The "functional group involved in the induction of phagocytosis by cells", when present in the gene introduction agent, induces a macrophagic cell having recognized the functional group to prey the gene introduction agent as a xenobiotic. It is preferable that the "functional group involved in the induction of phagocytosis by cells" exhibits a positive charge for recognition by a macrophagic cell, and examples of such a functional group include an amino group. In addition, the macrophagic cell can be different depending on a cell, a tissue and an organ targeted by the carrier for gene introduction and the gene introduction agent of the present invention, and for example, may be a macrophage, a microglia, a Kupffer cell or the like.

The "nano-particle" means a particulate substance having an average particle diameter of 10 nm to 1000 nm. The average particle diameter means an underwater particle diameter in a dispersion solvent. The underwater particle diameter can be measured by the previously known dynamic light scattering method. It is necessary that the "nano-particle" has such a size that the particle can be preyed by a macrophagic cell. A material and a shape of the "nano-particle" are not particularly limited, and it is preferable that the nano-particle is formed of a material having the certain hardness, and the material can maintain a shape in a living body over a certain period. In the present specification, the "nano-particle" is referred to as "nano-bead" or simply "particle" or "bead" in some cases.

The "substance capable of binding to a vector for gene introduction" may be a substance having a binding property with the vector for gene introduction, and is not particularly limited. As the "substance capable of binding to a vector for gene introduction", for example, when a vector is a virus vector, heparin or heparan sulfate can be used.

The "vector for gene introduction" is not particularly limited, and the vectors known in the art can be used, and for example, a virus vector can be used. The "virus vector" is not particularly limited, refers to a vector for introducing a gene utilizing the mechanism of infecting a cell of a virus or the mechanism of being maintained in a cell of a virus, and includes the known arbitrary virus vectors. In the present invention, for example, a sendaivirus vector, a lentivirus vector, a retrovirus vector, an adenovirus vector, an adeno-associated virus (AAV) vector and the like can be used. In the present invention, AAV1 to 8 types are preferably used, and AAV2 type is particularly preferably used.

### Effect of Invention

According to the gene introduction agent of the present invention, since the vector for gene introduction can be introduced into a desired site in a living body, it becomes possible to regiospecifically introduce a gene. In addition, since the gene introduction agent of the present invention is phagocytosed by a macrophagic cell, and is removed from a body in the course of time, safety of gene introduction can be enhanced.

According to the gene introduction agent of the present invention, stable binding between the carrier for gene introduction and the vector for gene introduction can be maintained in a tissue for a long term without destroying the vector for gene introduction. For this reason, only a cell which has been contacted with the gene introduction agent can be selectively infected with a virus, and it becomes possible to selectively and efficiently express a desired gene at a specified site in a living body. In addition, also in an in vitro system, since a specified site can be infected with a virus, a gene can be selectively expressed at a target site.

### Brief Description of Drawings

[Fig. 1] A schematic view illustrating a feature of the carrier for gene introduction and the gene introduction agent of the present invention.
[Fig. 2] A photograph showing a cultured hippocampus nerve cell after two weeks passed after addition of a gene introduction agent to which an adeno-associated virus vector with a GFP gene incorporated therein was bound (green: GFP, red: nano-bead).
[Fig. 3] A photograph of a rat brain after two weeks passed from injection of an aminated fluorescent magnetic bead, which was taken at ultrahigh resolution synchrotron radiation CT (white part shown with red arrow: aminated fluorescent magnetic bead).
[Fig. 4] A is an immunohistochemical stained image of a rat brain (red arrow site of Fig. 3) after two weeks passed from injection of an aminated fluorescent magnetic bead (violet: astrocyte, green: microglia, red: aminated fluorescent magnetic bead). A white arrow refers to a microglia which phagocytosed a large amount of an aminated fluorescent magnetic bead. B is an immunohistochemical stained image of a rat brain after two weeks passed from injection of a non-aminated fluorescent magnetic bead or aminated fluorescent magnetic bead (red: microglia, blue: fluorescent magnetic bead).
[Fig. 5] A photograph of a brain slice after four weeks passed from injection into a rat brain of a gene introduction agent to which an adeno-associated virus vector with a channelrhodopsin 2-GFP chimera gene incorporated therein was bound.
[Fig. 6] A chart when a nerve cell was pricked with a recording electrode, and irradiated with blue light, after four weeks passed from injection into a rat brain of a gene introduction agent to which an adeno-associated virus vector with a channelrhodopsin 2-GFP chimera gene incorporated therein was bound.
[Fig. 7] A photograph of a rat brain after two weeks passed from injection into a rat brain of a gene introduction agent to which an adeno-associated virus vector with a channelrhodopsin 2-GFP chimera gene incorporated therein was bound, which was taken with a fluorescent microscope (A; red: gene introduction agent, B; green: GFP, C; A+B multi fluorescent image).
[Fig. 8] A photograph of a rat brain after four weeks passed from direct injection into a rat brain of an adeno-associated virus vector with a channelrhodopsin 2-GFP chimera gene incorporated therein, which was taken with a fluorescent microscope.
[Fig. 9] Photographs of a culturing dish after a magnetic nano-wire bound with AAV with a GFP gene incorporated therein was placed on a cell, and the cell was cultured for three weeks, one of which was taken with an optical microscope (A) and another taken with a fluorescent microscope (B), and a photograph which was taken at ultrahigh resolution by synchrotron radiation CT, after injection of the magnetic nano-wire into a rat brain (C).

### Mode for Carrying Out the Invention

The present invention relates to a gene introduction agent characterized in that heparin or heparan sulfate is bound to the surface through a bond, and a vector for gene introduction is bound through heparin or heparan sulfate, a gene introduction method using the gene introduction agent, and a method for producing the gene introduction agent.

### 1. Carrier for gene introduction and Gene introduction agent

### [Gene introduction agent]

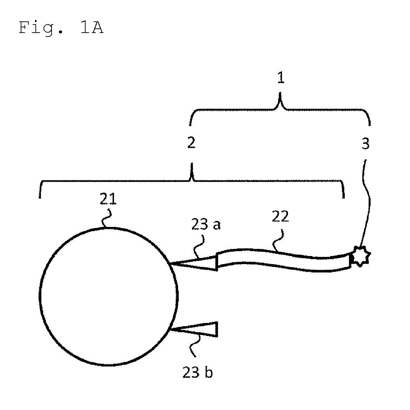

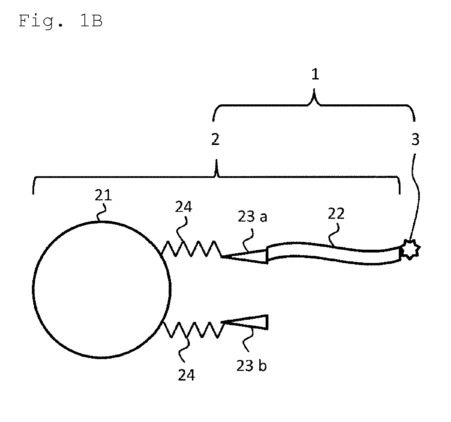

Fig. 1 schematically shows a feature of the carrier for gene introduction and the gene introduction agent of the present invention. The gene introduction agent shown with a symbol 1 in Fig. (A) comprises a carrier for gene introduction 2 and a vector for gene introduction 3 bound to this.

### [Vector for gene introduction]

The vector for gene introduction 3 is a vector in which a gene to be expressed in a target organ, tissue or cell (hereinafter, referred to as "target organ etc.") is incorporated. As the vector for gene introduction 3, for example, a virus vector can be used. As the virus vector, a sendaivirus vector, a lentivirus vector, a retrovirus vector, an adenovirus vector, an adeno-associated virus (AAV) vector and the like can be used. As the vector for gene introduction 3, AAV1 to 8 types are preferably used, and AAV2 type is particularly preferably used.

### [Carrier for gene introduction]

The carrier for gene introduction 2 comprises a nano-particle 21, and a substance capable of binding to a vector for gene introduction 22. The vector for gene introduction 3 is bound to the substance capable of binding to a vector for gene introduction 22. In addition, in the figures, one vector for gene introduction 3 binding to substance capable of binding to a vector for gene introduction 22 is shown for simplicity, but a plurality of vectors for gene introduction 3 may be bound to the substance capable of binding to a vector for gene introduction 22.

### [Nano-particle]

The nano-particle 21 may have a size of an average particle diameter of 10 nm to 1000 nm, and in order that the nano-particle is easily phagocytosed by a macrophagic cell, the nano-particle has a size of preferably a particle diameter of 150 nm to 300 nm, more preferably a particle diameter of 100 nm to 200 nm.

The nano-particle 21 may have magnetism. By applying magnetism to the nano-particle 21, the gene introduction agent 1 which has been introduced into a target organ etc. can be accumulated into a predetermined site by the external magnetic field. As the nano-particle 21 having magnetism, magnetic nano-particles which are known in the art, such as a magnetic nano-particle composed of a metal, a magnetic nano-particle composed of a polymer and the like can be used.

In the present invention, polymer-coated magnetic beads having the surface composed of a polymer described in Patent Literature 3 can be used without limitation, since they have an advantage that they are hardly aggregated. As the polymer, a polymer formed by a polymerization of a styrene monomer and a glycidyl methacrylate (GMA) monomer can be used. Since polystyrene obtained by a polymerization of styrene having strong hydrophobicity has the suitable hardness, it is preferable as a main constituent material of a bead. In addition, since a glycidyl group (epoxy group) of GMA reacts well with an amino group or the like, it can be substituted with an amino group or the like, or the substance capable of binding to a vector for gene introduction or a linker explained below can be bound to a group substituted with an amino group or the like.

The monomer used for forming a polymer which coats a magnetic bead is not particularly limited, as far as it is a monomer having a radical polymerizable functional group. Examples of the monomer include aromatic vinyl compounds such as styrene, α-methylstyrene, o-vinyltoluene, m-vinyltoluene, p-vinyltoluene, divinylbenzene and the like; unsaturated carboxylic acids such as (meth)acrylic acid, crotonic acid and the like; (meth)acrylates such as methyl (meth)acrylate, ethyl (meth)acrylate, n-propyl (meth)acrylate, i-propyl (meth)acrylate, n-butyl (meth)acrylate, t-butyl (meth)acrylate, n-hexyl (meth)acrylate, 2-ethylhexyl (meth)acrylate, (poly)ethylene glycol di(meth)acrylate, (poly)propylene glycol di(meth)acrylate, trimethylolpropane tri(meth)acrylate, and glycidyl (meth)acrylate; vinyl cyanide compounds such as (meth)acrylonitrile, vinylidene cyanide and the like; and halogenated vinyl compounds such as vinyl chloride, vinylidene chloride, vinyl fluoride, vinylidene fluoride, tetrafluoroethylene and the like. Among these monomers, aromatic vinyl compounds, and (meth)acrylates are particularly preferable. In addition, these monomers can be used alone, or two or more can be used by mixing them. It is preferable that at least one of these monomers has the surface coated with a polymer after polymer formulation, having functional groups 23a, 23b which will be explained below, or a functional group replaceable with functional groups 23a, 23b.

### [Substance capable of binding to vector for gene introduction]

The substance capable of binding to a vector for gene introduction 22 is not particularly limited, and can be any substance having the ability to bind to the vector for gene introduction 3. As the substance capable of binding to a vector for gene introduction 22, for example, in the case where the vector is a virus vector, heparin or heparan sulfate may be used.

### [Functional group]

The nano-particle 21 has functional groups 23a, 23b involved in the induction of phagocytosis by cells. The functional groups 23a, 23b are a functional group which is recognized by a macrophagic cell, and induces predation by the cell of the gene introduction agent 1 as a foreign substance in a living body, and are, for example, an amino group or the like (see Non-Patent Literatures 3 and 4). Functional groups 23a, 23b may be the same or different. In the figures, for simplicity, each one of functional groups 23a, 23b is shown, but the nano-particle 21 has a plurality of functional groups 23a, 23b.

The substance capable of binding to a vector for gene introduction 22 is bound to the surface of the nano-particle 21 through a functional group 23a. A bond between the functional group 23a and the substance capable of binding to a vector for gene introduction 22 may be a chemical bond or a physical bond. Herein, the chemical bond refers to binding between atoms in a molecule or a crystal and is classified into a covalent bond, an ionic bond, a metal bond, and a coordinate bond. In the present invention, examples include a bond through an amino group, a thiol group, or an active ester group, a sulfide bond, and a disulfide bond, without limitation. The physical bond refers to the state where a substance is adsorbed with the Van der Waals force or static electricity.

As a result of the chemical bond or the physical bond with the substance capable of binding to a vector for gene introduction 22, the functional group 23a may have lost the ability to induce phagocytosis by cells. On the other hand, the functional group 23b remains unbound to the substance capable of binding to a vector for gene introduction 22. For this reason, the functional group 23b maintains the ability to induce phagocytosis by cells.

### [Effect of a gene introduction agent]

In the gene introduction agent 1, the substance capable of binding to a vector for gene introduction 22 is bound to the nano-particle 21 through the functional group 23a, and the vector for gene introduction 3 is further bound to the substance capable of binding to a vector for gene introduction 22 to retain the vector for gene introduction 3 on the nano-particle 21. For this reason, when the gene introduction agent 1 is introduced into a target organ etc., the vector for gene introduction 3 retained on the nano-particle 21 remains at an introduction site without diffusion. Therefore, in gene introduction with the gene introduction agent 1, it is possible to express an objective gene only at an introduction site of the gene introduction agent 1 of a target organ etc.

Further, in the gene introduction agent 1, the functional group 23b which remains unbound to the substance capable of binding to a vector for gene introduction 22 induces phagocytosis of the gene introduction agent 1 by cells. Therefore, in gene introduction by the gene introduction agent 1, the gene introduction agent 1 after expression of an objective gene at a specific site of a target organ etc. is made to be phagocytosed by a macrophagic cell, thereby, it can be removed from a body.

In order to promote phagocytosis of the gene introduction agent 1 by a macrophagic cell, the same functional group (amino group etc.) as the functional group 23a or the functional group 23b can be bound to the substance capable of binding to a vector for gene introduction 22 in advance.

### [Linker]

The functional groups 23a, 23b may exist on the nano-particle 21 through a linker 24, as shown in Fig. (B). The linker 24 is not particularly limited, and a hydrophilic molecule such as ethylene glycol (EG) having a unit of "-CH₂-CH₂-O-" in a molecular structure, propylene glycol having a unit of "-CH(CH₃)-CH₂-O-" in a molecular structure, and butylene glycol having a unit of "-CH(CH₃)-CH₂-CH₂-O-" in a molecular structure can be used. By introducing the linker 24, it is possible to improve wettability of the nano-particle 21, suppress aggregation of the nano-particle 21, and enhance solvent dispersibility of the gene introduction agent 1.

An ethylene glycol chain (EG chain) is represented by "-(CH₂-CH₂-O)m-" (m is an integer indicating a polymerization degree), and an EG chain in which m is 2 or more is called polyethylene glycol chain (PEG chain) in some cases. As the linker 24, a compound containing an EG chain in a molecular structure, and having a reactive functional group such as an epoxy group, amino group, a carboxyl group, a maleimide group, a hydroxyl group, a succinimide group, an azido group, an alkynyl group, and a diazirine group on an end is preferable. As the linker 24, an ethylene glycol-based compound having an epoxy group such as a glycidyl group on both ends of a molecule, in which m is 1 or more and 5 or less, is preferable, and ethylene glycol diglycidyl ether (EGDE) having glycidyl ether on both ends of a molecule, in which m is 1, is particularly preferable.

### 2. Method for producing a carrier for gene introduction or a gene introduction agent

### (1) Method for producing a carrier for gene introduction

### [Step of binding a functional group]

In order to prepare the carrier for gene introduction 2, first, treatment of reacting a compound having functional groups 23a, 23b which induce phagocytosis by cells with the nano-particle 21 to bind functional groups 23a, 23b to the surface of the nano-particle 21 is performed. The case where functional groups 23a, 23b are an amino group, and the compound having functional groups 23a, 23b is an amino group donating compound will be explained below as an example.

The amino group donating compound is not particularly limited, and for example, ammonia, a basic amino acid such as lysine and arginine, and a basic polypeptide such as polylysine and polyarginine can be used. The reaction condition such as a solvent, a temperature, a reaction time and the like which is adopted in treatment of binding an amino group can be appropriately set by a person skilled in the art, depending on the nano-particle 21 and/or the amino group donating compound.

In addition, the present step can be omitted in some cases, in the case where a nano-bead having an amino group even when the present treatment is not conducted, such as a nano-bead composed of a polymer having an amino group in a main chain or a side chain is used as the nano-particle 21.

### [Step of binding a substance capable of binding to a vector for gene introduction]

Then, treatment of binding the substance capable of binding to a vector for gene introduction 22 to the nano-particle 21 through the functional group 23a is performed. Thereby, the carrier for gene introduction 2 is obtained, as the nano-particle 21 to which functional groups 23a, 23b and the substance capable of binding to a vector for gene introduction 22 are added. Hereinafter, the case where the substance capable of binding to a vector for gene introduction 22 is heparin will be explained as an example.

For heparin binding treatment, the known heparin binding procedure can be used, and heparin can be bound, for example, by chemical treatment using a commercially available reagent which binds heparin to an organic compound or the like. By such treatment, heparin is bound to the surface of the nano-particle 21 through an amino group (functional group 23a). In place of heparin, heparan sulfate may be used. Alternatively, the nano-particle 21 and heparin can be bound utilizing an active ester group.

In the case where heparin is bound through an amino group on the surface of the nano-particle 21, as shown in the following formula 1, an amino group (NH₂) on the surface of the nano-particle 21 and a formyl group (CHO) in heparin are bound to form an imine (R-N=CH-R), and then, such an imine is reduced to an amine.
[Chemical formula 1]

R'-NH₂ + R"-CHO → R'-N=CH-R" → R'-NH-CH₂-R" Formula 1

In addition, the formation of an imine is an equilibrium reaction (first stage of the formula 1), and reduction of an imine (second stage of the formula 1) is an irreversible reaction.

Binding between an amino group on the surface of the nano-particle 21 and heparin does not occur for all amino groups present on the surface of the nano-particle 21, due to a molecular size of heparin and an efficiency of a reaction between an amino group and a formyl group. For this reason, an amino group (functional group 23a) which forms a bond with heparin, and an amino group (functional group 23b) which remains unbound to heparin are present on the surface of the nano-particle 21 after a binding reaction, at the certain ratio. This also applies to the case where functional groups 23a, 23b are a functional group other than an amino group, and the substance capable of binding to a vector for gene introduction 22 is a substance other than heparin. The ratio between the functional group 23a which forms a bond with the substance capable of binding to a vector for gene introduction 22, and the functional group 23b which remains unbound on the surface of the nano-particle 21 after the present step varies depending on the kind of the substance capable of binding to a vector for gene introduction 22 and the reaction condition, and is around 5 : 5 to 1 : 9.

### (2) Method for producing a gene introduction agent

### [Step of binding a vector for gene introduction]

Treatment of binding the vector for gene introduction 3 to the carrier for gene introduction 2 is performed. For example, the carrier for gene introduction 2 with an amino group and heparin added thereto is placed in a column, and a virus-partially purified sample is added, thereby, the gene introduction agent 1 in which the virus vector is bound to heparin can be obtained. In addition, construction and purification of the vector for gene introduction 3 can be performed by using commercially available reagents or the like by the known methods. Since in the carrier for gene introduction 2, an arbitrarily selected vector for gene introduction 3 with an objective gene incorporated therein can be bound, it is possible to easily prepare a desired gene introduction agent 1, depending on an object of gene introduction and the kind of a target cell, etc.

In addition, in one aspect of the present invention, in addition to the vector for gene introduction 3, optionally, an adhesive molecule (laminin etc.) or a humoral factor (elicitor, nutritional factor or stimulating factor such as chemokine etc.) having the ability to bind to heparin or a heparan sulfate is added by mixing with the vector for gene introduction 3, and those molecules may be bound to the gene introduction agent 1 through heparin or heparan sulfate.

For example, by inducing or activating a specified cell using the humoral factor, or by adhering a cell utilizing an adhesion factor, an opportunity to contact the target cell and the virus vector can be increased to improve an efficiency of infection with the virus vector.

In addition, even in the case where a molecule having the ability to bind to heparin or heparan sulfate is not bound with the virus vector, since a heparin binding protein possessed by a cell or a tissue being a target of gene introduction binds to a heparin residue remaining on the surface of the nano-particle 21, an efficiency of infection with the virus vector becomes higher than that of the case of only the nano-particle 21.

In the method for producing the carrier for gene introduction or the gene introduction agent of the present invention, as the functional group 23a, in place of an amino group, a thiol group can be used in some cases. In this case, in place of the amino group donating compound, a thiol group donating compound is used. The thiol group donating compound is not limited, but for example, cysteine, carboxy disulfide, a straight-chain thiol reagent, an aromatic ring-type dithiol reagent or the like can be used. The substance capable of binding to a vector for gene introduction 22 is bound to the surface of the nano-particle 21 by a sulfide bond or a disulfide bond through a thiol group.

### 3. Method for introducing a gene into a target cell, a target tissue or a target organ

In the present invention, the "target cell" is not particularly limited, but may be any cell. For example, cells of a tissue or an organ of a living body, and various cultured cells can be included as the target cell. In addition, in the present invention, the "target tissue" and the "target organ" are not particularly limited, but may be any tissue or organ. For example, brain, liver, heart, kidney, muscle, lung, ovary, uterus, testis, digestive tract, and blood vessel can be included as the target organ, without any limitation.

The gene introduction method of the present invention can be used not only for in vitro, but also in vivo and ex vivo gene introduction. Therefore, the present invention, as other aspect, can provide a gene therapy method comprising a step of performing gene introduction by the gene introduction method of the present invention. The method of the present invention can be applied to a human and a non-human mammal (e.g. rodent such as mouse, rat etc.). A gene to be introduced in gene therapy is not particularly limited, but examples include a disease causative gene and a photoresponsive gene. For example, when the photoresponsive gene is introduced into the target organ etc. using the present invention, the function of an organ can be controlled by light.

Since the gene introduction agent 1 uses the nano-particle 21, a gene can be locally introduced into a desired position by injection, utilizing a syringe or a catheter.

When the gene introduction agent 1 is introduced into the target organ etc., a cell which has contacted with the vector for gene introduction 3 bound to the surface of the nano-particle 21 ingests the vector for gene introduction 3 into the cell, and becomes to express a desired gene.

In the gene introduction method of the present invention, since the gene introduction agent 1 which has been locally injected can be retained while localized, that is, the vector for gene introduction 3 can be localized at a desired site for a long term, a gene expression efficiency is improved, as compared with the previous gene introduction method by which the introduced vector for gene introduction 3 is diffused. In addition, in the gene introduction method of the present invention, diffusion of the vector for gene introduction 3 to a site which is not a desired site (site outside an object) is extremely smaller as compared with the previous gene introduction method, and therefore gene expression at a site outside an object can be suppressed, and the unpreferable action can be reduced.

Further, when the nano-particle 21 is a magnetic particle, by applying the magnetic field from the outside, the introduced gene introduction agent 1 can be moved to a desired position, or can be localized and retained at a desired position (injected site). In order to apply the magnetic field from the outside, a magnetic field controlling apparatus composed of an inducing needle, a controlling portion, and a magnet which is disclosed, for example, in Patent Literature 1 may be used. Herein, the magnet is a magnetic field generator which generates the magnetic field inducing a magnetic particle, and the inducing needle is a needle which enhances the magnetic flux density by the magnetic field generated from the magnet, at a tip portion. In addition, the controlling portion is a controlling portion which controls the magnetic field between the magnet and the inducing needle.

In the case where the nano-particle 21 is the magnetic particle, since the gene introduction agent 1 can be detected by X-ray CT, it becomes possible to confirm that the gene introduction agent 1 has been correctly introduced into an objective place, and that the gene introduction agent 1 has been removed from the introduced place.

### 4. Removal of a gene introduction agent by a macrophagic cell

When the time passed after administration to a living body, the gene introduction agent 1 undergoes phagocytosis by a macrophagic cell, and is removed from an administration site. It is considered that this is because the functional group 22b functions as a phagocytosis signal for the macrophagic cell. In addition, by dissolution of the substance capable of binding to a vector for gene introduction 22 after uptake of the vector for gene introduction 3 into a cell, there is a possibility that the functional group 22a exposed on the surface of the nano-particle 21 also functions as a phagocytosis signal.

Further, in order to promote phagocytosis of the gene introduction agent 1 by the macrophagic cell, the same functional group (amino group etc.) as the functional group 23a or the functional group 23b may be bound to the substance capable of binding to a vector for gene introduction 22.

Since the gene introduction agent 1 is removed from a body with passage of the time after administration to a living body, the gene introduction method of the present invention has higher safety as compared with the previous gene introduction method.

The present invention will be illustrated in more detail below by way of Examples, but these Examples do not limit the present invention.

### Examples

### 1. Preparation of a carrier for gene introduction (1) Preparation of a magnetic bead (FG bead)

As a core of a magnetic bead, a ferrite particle having an average particle diameter of approximately 40 nm was selected.
0.5 mmol of 10-undecenoic acid was added to a dispersion of the ferrite particle to completely hydrophobize the surface of the ferrite particle. Further, 0.47 g of a 60% aqueous solution of Emulgen 1150S-60 (Kao Corporation) which is a nonionic surfactant was added, and the resultant was applied to an ultrasound-treated to, thereby, the ferrite particle was again converted to hydrophilic. As a result, the ferrite particle could be dispersed in an aqueous solution at a particle diameter of approximately 90 nm.

Then, into the dispersion of the ferrite particle were added a styrene monomer, a glycidyl methacrylate (GMA) monomer, and a divinylbenzene (DVB) monomer at 2.7 g, 0.3 g and 0.04 g, respectively, and water was added to a total weight of 240 g. Stirring was performed in a constant temperature tank at 70°C, 10 g of an aqueous solution obtained by dissolving 60 mg of V-50 (Wako Pure Chemical Industries, Ltd.) being a polymerization initiator was added after 20 minutes, and a polymerization reaction was performed. Two hours after polymerization initiation, 0.3 g of GMA was post-added, and a polymerization reaction was performed for 16 hours from that time point. A magnetic bead (FG bead) after the reaction was recovered by centrifugation (20,000 G, 20 min), and washing with 50 ml of ultrapure water was performed three times. After a washing operation, the bead was dispersed in 10 ml of ultrapure water, and dialysis treatment against 2L of ultrapure water was performed three times. An average particle diameter of the magnetic bead was 200 nm.

### (2) Introduction of a linker and an amino group into a magnetic bead

### (i) Preparation of a linker-bound magnetic bead (EGDE bead)

1.0 g of a magnetic bead (FG bead) was dispersed into 100 ml of an aqueous ammonia solution (pH 11.0), and a reaction was performed at 70°C for 24 hours with stirring. After the reaction, the magnetic bead was recovered by centrifugation (20,000 G, 20 min), and washing with 50 ml of ultrapure water was performed three times. After a washing operation, the bead was dispersed in 10 ml of ultrapure water, and dialysis treatment against 2 L of ultrapure water was performed three times.

Subsequently, 0.2 g of the resulting magnetic bead was dispersed in 36 ml of an aqueous solution (pH 11.0) of ethylene glycol diglycidyl ether (EGDE), and a reaction was performed at 30°C for 24 hours with stirring. After the reaction, the magnetic bead (EGDE bead) with EGDE bound thereto as a linker was recovered by centrifugation (20,000 G, 20 min), and washing with 50 ml of ultrapure water was performed three times. After a washing operation, the bead was dispersed in 10 ml of ultrapure water, and dialysis treatment against 2 L of ultrapure water was performed three times.

### (ii) Preparation of an aminated magnetic bead (EGDEN bead)

1.0 g of the linker-bound magnetic bead (EGDE bead) was dispersed into 45 ml of an aqueous ammonia solution (pH 11.0), and a reaction was performed at 70°C for 24 hours with stirring. The aminated magnetic bead (EGDEN bead) after the reaction was recovered by centrifugation (20,000 G, 20 min), and washing with 50 ml of ultrapure water was performed three times. After a washing operation, the bead was dispersed in 10 ml of ultrapure water, and dialysis treatment against 2 L of ultrapure water was performed three times.

The aminated magnetic bead (EGDEN bead) was made to absorb a fluorescent europium complex onto a polymer layer, according to the method described in Patent Literature 5.

### (3) Preparation of a carrier for gene introduction

Heparin was bound to the aminated magnetic bead (EGDEN bead) to prepare a carrier for gene introduction. After 1.0 mg of the aminated magnetic bead was dispersed in PBS, and the resultant was centrifuged, the supernatant was removed. 445 µL of PBS, 50 µL of a 30 mg/mL heparin solution (PBS), and 5.0 µL of a 30 mg/mL NaBH₃CN solution (PBS) were added, respectively, to disperse the bead in the reaction solution. The reaction solution was stirred at room temperature for 10 days using a mixer. After the reaction, the bead was recovered by centrifugation, the supernatant was removed, and ultrapure water was added to wash the bead, to obtain a carrier for gene introduction.

In the carrier for gene introduction, the existence ratio between an amino group forming a bond with heparin, and a free amino group not forming the bond was presumed that the free amino group is at least 50%, and maximally 90% or more, on the premise of the aforementioned reaction condition.

### 2. In vitro gene introduction

### (1) Preparation of a gene introduction agent

An adeno-associated virus vector (AAV) with a GFP gene incorporated therein was added to the carrier for gene introduction which had been prepared according to the 1, to bind AAV to heparin, to obtain a gene introduction agent. The gene introduction agent emits red (615 nm) fluorescence by an enclosed fluorescent europium complex.

In addition, replication and proliferation of AAV were performed using the AAV-2 helper-free expression system (Cell Biolabs, Inc.) according to an instruction manual of the product.

### (2) Introduction of a gene into a target cell

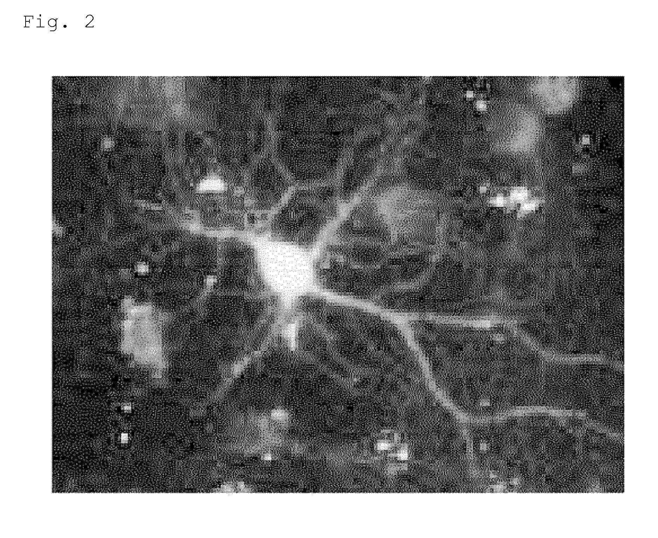

The gene introduction agent prepared in the (1) was added to a cultured hippocampus nerve cell. When the cultured hippocampus nerve cell was observed using a fluorescent microscope (Fig. 2) after three weeks from addition, the nerve cell which was contacted with the gene introduction agent was infected with AAV to express a GFP gene, and emitted green fluorescence from the whole cell. The gene introduction agent emitted red fluorescence. From Fig. 2, it is clear that AAV is little eliminated from the bead, and is bound to the bead in the state where AAV had the activity.

### 3. Effect generated by in vivo introduction of an amino group

### (1) Preparation of an aminated magnetic bead

According to the 1 (1) and (2), an aminated magnetic bead (EGDEN bead) was prepared.

### (2) Localization of an aminated magnetic bead in a target site

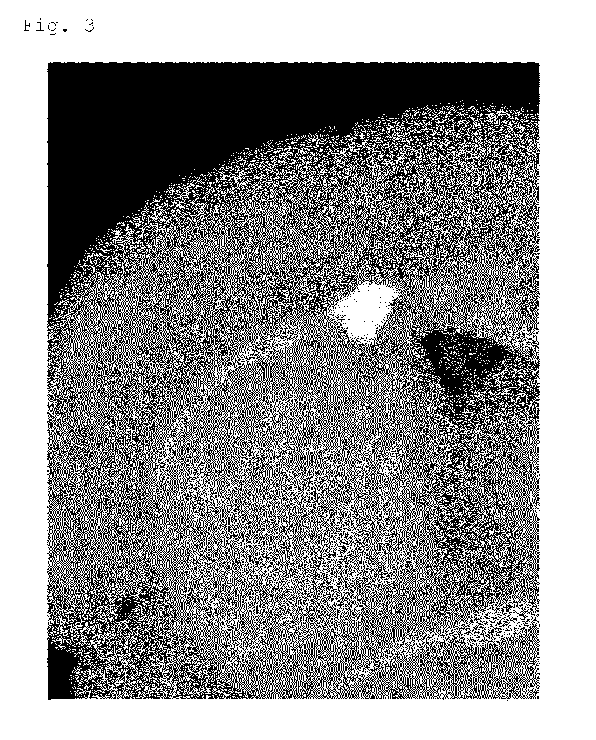

The aminated magnetic bead was injected into a rat brain. After two weeks from injection, when the brain was photographed at high resolution using synchrotron radiation CT (Fig. 3), an appearance that the injected bead was localized at a part of the brain was observed (Fig. 3, white part is bead).

### (3) Phagocytosis of an aminated magnetic bead by microglia

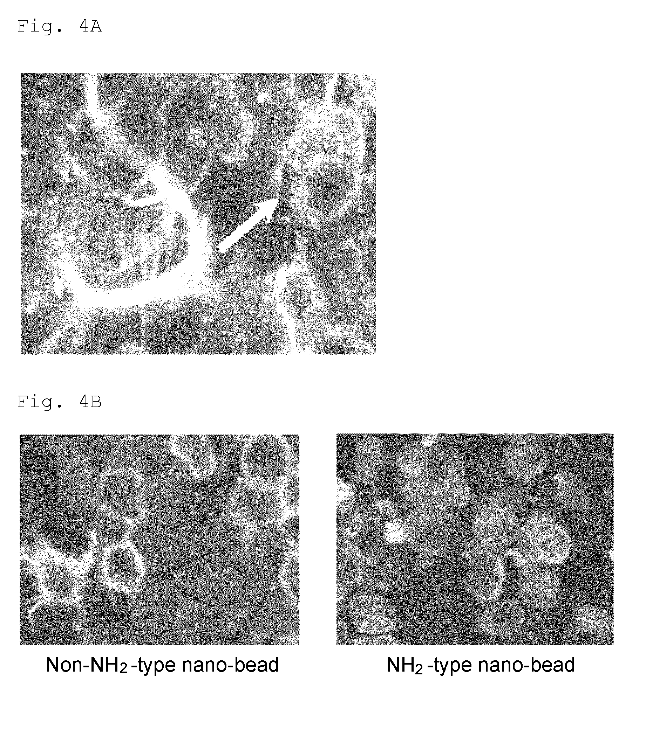

(i) Further, the same site was subjected to immunohistochemical staining, and observed using a fluorescent microscope (Fig. 4A). Using an anti-GFAP antibody (Sigma, ×1000) as a primary antibody, and an antimouse antibody (Sigma, ×500) as a secondary antibody, a glial fiber acidic protein (GFAP) was stained, and an astroglia cell was identified. Separately, a cell membrane of a microglia was stained green by a selective staining method using lectin. The aminated magnetic nano-bead emitted red fluorescence by a contained europium complex. From Fig. 4A, it is seen that a microglia phagocytosed a large amount of the bead (Fig. 4A, white arrow).
(ii) The aminated magnetic bead prepared in the (1) or a non-aminated magnetic bead (FG bead) was injected into a rat brain. According to the protocol as that of the (i), after two weeks from injection, the sample was subjected to immunohistochemical staining, and observed using a fluorescent microscope (Fig. 4B). The nano-bead is shown with blue, and a cell membrane of a microglia is shown with red. A majority of the aminated magnetic beads (NH2-type nano-bead) were phagocytosed by a microglia (macrophage), and for this reason, a microglial cell was swollen up. On the other hand, in the case of the non-aminated magnetic bead (non-NH2-type nano-bead), a majority of the beads remained at an injection portion, and an amount of the bead in the microglial cell was smaller as compared with that of the aminated magnetic bead. Many of the aminated magnetic beads were phagocytosed in four weeks, and a majority of nano-beads were removed from an injection location after 8 to 12 weeks (data are not shown).

From the experiment, it is clear that the magnetic bead having an amino group is phagocytosed and removed by a microglia after a certain term. In addition, the aminated magnetic bead has a high pH, but even when injected into a brain, there was no neurotoxicity, and a remarkable inflammation reaction was not induced.

### 4. In vivo gene introduction

### (1) Preparation of a gene introduction agent

To the carrier for gene introduction prepared according to the 1 was added AAV with a chimera gene (channelrhodopsin 2-GFP) of a photoresponsive ion channel (channelrhodopsin 2) and GFP incorporated therein, to bind AAV to the carrier for gene introduction, to thereby obtain a gene introduction agent.

### (2) Introduction of a gene into a target site

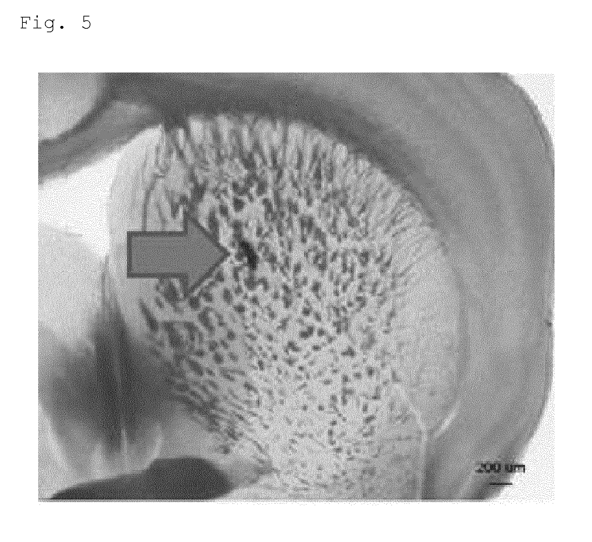

The gene introduction agent prepared in the (1) was injected into a rat brain. After four weeks from injection, the rat was experimentally killed, and a brain slice was made (Fig. 5). An injection site is shown with a blue arrow. From Fig. 5, it is clear that the gene is expressed only at a gene introduction agent injection site, and the virus is not diffused to other site.

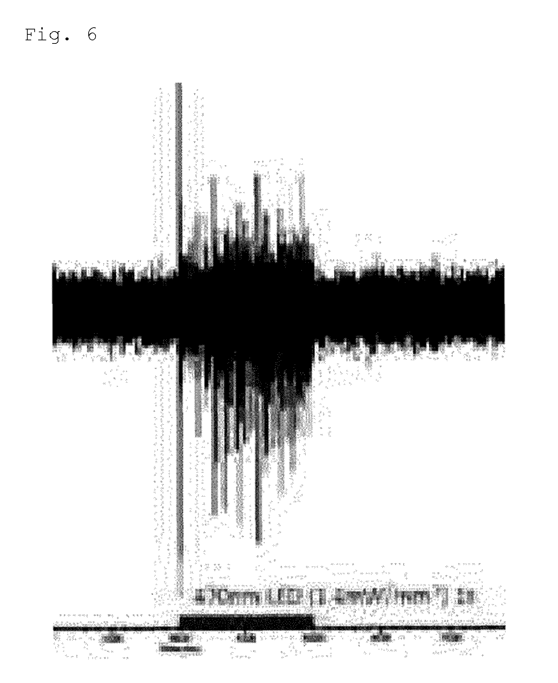

Further, when the nerve cell at a portion where a gene introduction agent was injectioned was pricked with a recording electrode, and then blue light (470 nm) was irradiated, the nerve activity was induced (Fig. 6). That is, it is clear that AAV having the physiological activity is mounted in the gene introduction agent.

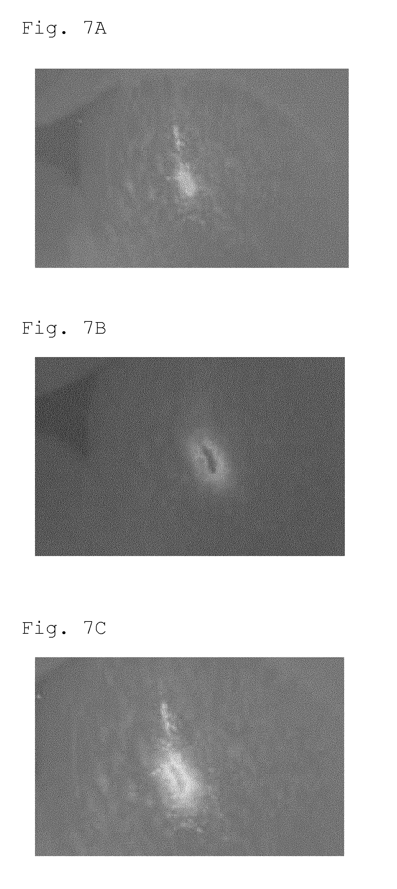

In addition, separately, when the gene introduction agent prepared in the (1) was injected into a rat brain, and the brain was photographed at high resolution using a fluorescent microscope after two weeks (Fig. 7), the gene was expressed only at a portion where a gene introduction agent was intentioned (red: gene introduction agent, green: GFP). Punctate red fluorescent spots are seen at a periphery of green fluorescence, and the red fluorescent spot is the gene introduction agent phagocytosed by a microglia. That is, the gene introduction agent which has finished a role is removed by a microglia.

### 5. Introduction of a thiol group into a magnetic nano-wire and binding of heparin, as well as impartation of the magnetic nano-wire to a cell and administration of the magnetic nano-wire to a rat (Reference Example)

### (1) Method for binding heparin to a metal nano-wire

Introduction of a thiol group into a nano-wire and binding of heparin to a nano-wire were performed according to the following protocol.
(i) Washing of wire and polymer with acetone
(ii) Preparation of a heparin reaction reagent
   0.05M MES buffer (pH 5.4) (when becomes in an acidic region, a pH is adjusted with NaOH)
   10 mg Heparin
   Incubation in the presence of 15 mg EDC (1-ethyl-3-(3-dimethylamino-propyl)carbodiimide) for 15 minutes; activation of heparin 9 mg NHS (N-hydroxysuccinimide)
(iii) Silane coupling (only inorganic substrate such as SUS)
(iv) A wire and a polymer were added to a solution of (ii), and this was incubated at room temperature for 24 hours with stirring.
(v) Washing

Washing was performed with 0.05 M MES (2-morpholinoethanesulfonic acid, monohydrate), PBS (2 hours), 4 M NaCl (2 hours), and distilled water (2 hours × twice) in this order. Herein, a composition of PBS is KCl 0.2 g/L, KH₂PO₄ 0.2 g/L, Na₂HPO₄·12H₂O 2.9 g/L, NaCl 8 g/L.

### (2)Impartation of a nano-wire to a cell

Heparin was bound to a magnetic nano-wire (photograph is stainless extra-fine wire), and further, AAV was loaded to infect a cultured cell with a virus vector. 293 Cells were cultured on the whole surface of a culturing dish, a net of the magnetic nano-wire loaded with AAV was placed thereon, and this was cultured for 3 weeks. Since a vector encoding a gene of a fluorescent protein GFP is incorporated into AAV, a cell which has been infected to express GFP emits green fluorescence.

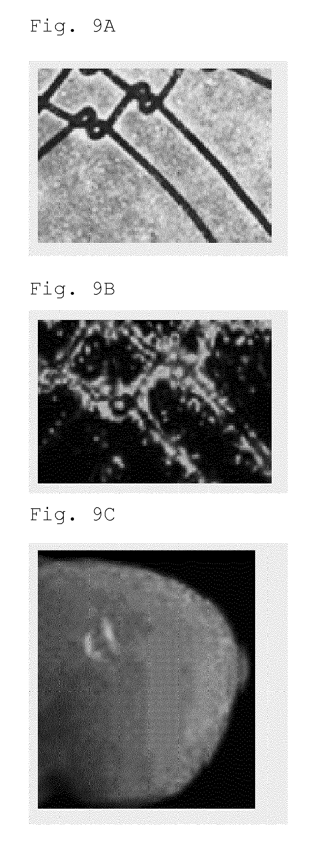

As in a photograph, only a cell which has contacted with the magnetic nano-wire net emits green fluorescence (Figs. 9A and 9B). This explicitly shows that AAV little secedes from the magnetic nano-wire net, and AAV is bound to the magnetic nano-wire in the state where it has the activity.

### (3) Administration of a nano-wire to a rat brain

The magnetic nano-wires prepared in the (1) and (2) were injected into a rat brain. After 2 weeks from injection, when the brain was photographed at high resolution using synchrotron radiation CT (Fig. 9C), an appearance that the injected magnetic nano-wire was localized at a part of the brain was observed (Fig. 9C, white portion is magnetic nano-wire).

### 6. In vivo gene introduction without using a nano-particle (Comparative Example)

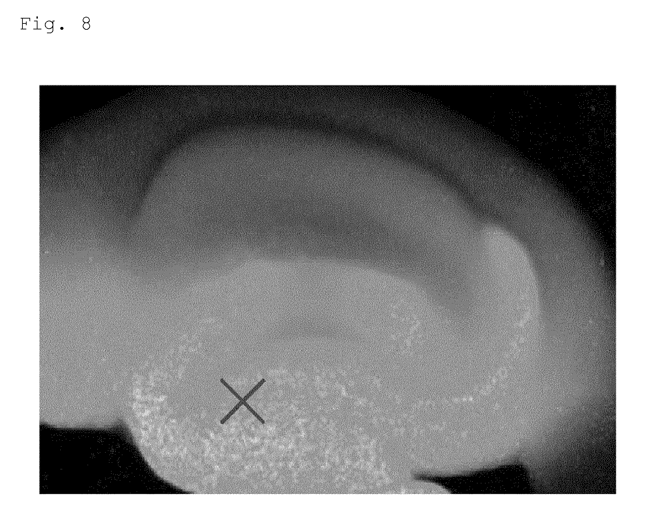

After a rat was subjected to general anesthesia, a head of the rat was fixed at a stereotaxic brain operation apparatus, and AAV with a GFP gene incorporated therein was simply injected into a rat brain (Fig. 8 place of symbol ×) using a micromanipulator. After four weeks, the rat was killed by deep anesthesia, a hippocampus region was cut out, and observed with a fluorescent microscope (Fig. 8). For incorporating a GFP gene into AAV, as in the 2 (1), a product manufactured by Cell Biolabs, Inc. was used. AAV was diffused to not only a portion injected with the vector but also a wide range in the brain, and the GFP gene was expressed. In this way, in the case where a virus is injected simply, since a gene is expressed at a portion outside an object, there is a risk of the serious side effect depending on a gene to be introduced.

### 7. Preparation of a gene introduction agent in which heparin is directly bound to the nano-particle surface

### (Comparative Example)

The present inventors tried to directly bind heparin to the surface of the aminated magnetic bead (EGDEN bead) prepared in the 1 without through an amino group. However, a sufficient amount of heparin could not be bound without breaking the magnetic bead (under the mild reaction condition, a binding amount of heparin was small, and under the extreme reaction condition, the magnetic bead was broken, or a fluorescent complex was flown out) (data are not shown).

### 8. Safety test

### (1) In vitro test

To the carrier for gene introduction prepared according to the 1 was added an adeno-associated virus vector (AAV) with an EGFP gene incorporated therein to bind AAV to heparin, to obtain a gene introduction agent.

Cultured hippocampus nerve cells were seeded on a 24-well plate (4 × 10⁴ cells/well), and cultured. On 8th day of culturing, the gene introduction agent was added to a culturing liquid (addition concentration 2 µg/ml). On 21st day of culturing, cells were fixed with 4% PFA, and a neurofilament was observed using an immunohistochemical procedure.

The fiber length of the neurofilament was measured concerning a group of addition of the carrier for gene introduction and a non-addition condition group. As a result, a significant difference of the fiber length was not recognized between both experimental groups, and it was made clear that the carrier for gene introduction of the present invention does not exhibit cytotoxicity.

### (2) In vivo test

To the carrier for gene introduction prepared in the 1 was added AAV with a photoresponsive ion channel (channelrhodopsin 2) introduced therein, to bind AAV to the carrier for gene introduction, to obtain a gene introduction agent.

The gene introduction agent was injected into a rat brain. After two weeks from injection, the presence or absence of brain edema was evaluated using synchrotron radiation phase difference CT.

Further, a nerve cell at a portion of injection of the gene introduction agent was pricked with a recording electrode, and excitation of the nerve cell when irradiated with blue light (470 nm) was measured electrophysiologically to, thereby, confirm gene expression of the photoresponsive ion channel.

As a result, at a portion of injection of the gene introduction agent, gene expression of the photoresponsive ion channel was confirmed, while brain edema was not recognized, or was recognized extremely slightly. From this, it was made clear that the gene introduction agent of the present invention has no toxicity on a living body.

### Industrial Applicability

According to the gene introduction agent of the present invention, since a vector for gene expression use can be introduced into a desired position in a living body, and when a certain term has passed, the gene introduction agent is removed from a body, a gene can be introduced into a specified position of a living body safely and freely. Therefore, according to the gene introduction agent of the present invention, for example, in treatment requiring an operation in the current gene introduction technique such as gene therapy on a brain disease and the like, a new method of treatment having a small burden on a patient can be provided.

### Description of the Reference Numbers

1: gene introduction agent
2: carrier for gene introduction
3: vector for gene introduction
21: nano-particle
22: substance capable of binding to a vector for gene introduction
23a, 23b: functional group
24: linker

## Claims

1. A carrier for gene introduction comprising a nano-particle and a substance capable of binding to a vector for gene introduction, wherein
the carrier has functional groups involved in the induction of phagocytosis by cells,
the substance capable of binding to a vector for gene introduction can bind to the surface of the nano-particle through some of the functional groups, and
another some of the functional groups remain unbound to the substance capable of binding to a vector for gene introduction.

2. A gene introduction agent, **characterized in that** a vector for gene introduction is bound to the substance capable of binding to a vector for gene introduction in the carrier for gene introduction according to claim 1.

3. The gene introduction agent according to claim 2, wherein the vector for gene introduction is one or more selected from the group consisting of a sendaivirus vector, a lentivirus vector, a retrovirus vector, an adenovirus vector and an adeno-associated virus vector.

4. The gene introduction agent according to claim 2 or 3, wherein the functional group has a positive charge.

5. The gene introduction agent according to any one of claims 2 to 4, wherein the functional group is an amino group.

6. The gene introduction agent according to any one of claims 2 to 5, wherein an average particle diameter of the nano-particle is 10 nm to 1000 nm.

7. The gene introduction agent according to any one of claims 2 to 6, wherein the substance capable of binding to a vector for gene introduction is heparin and/or heparan sulfate.

8. The gene introduction agent according to any one of claims 2 to 7, wherein the nano-particle has magnetism.

9. A method for introducing a gene into a cell comprising using a gene introduction agent which comprises a nano-particle, a vector for gene introduction, and a substance capable of binding to a vector for gene introduction, wherein
the agent has functional groups involved in the induction of phagocytosis by cells,
the substance capable of binding to a vector for gene introduction can bind to the surface of the nano-particle through some of the functional groups,
another some of the functional groups remain unbound to the substance capable of binding to a vector for gene introduction, and
the vector for gene introduction is bound to the substance capable of binding to a vector for gene introduction.

10. A method for introducing a gene into a cell, comprising a procedure of binding a vector for gene introduction to a substance capable of binding to a vector for gene introduction in a carrier for gene introduction comprising a nano-particle and the substance capable of binding to a vector for gene introduction, wherein
the carrier has functional groups involved in the induction of phagocytosis by cells,
the substance capable of binding to a vector for gene introduction can bind to the surface of the nano-particle through some of the functional groups, and
another some of the functional groups remain unbound to the substance capable of binding to a vector for gene introduction,
to prepare a gene introduction agent.

11. The gene introduction method according to claim 9 or 10, comprising a procedure of guiding the gene introduction agent to a target cell by injection.

12. The gene introduction method according to claim 11, wherein the nano-particle has magnetism, and the method comprises a procedure of applying the magnetic field to the gene introduction agent to retain it at an injection site.

13. A method for producing a carrier for gene introduction comprising a nano-particle having functional groups involved in the induction of phagocytosis by cells on the surface and a substance capable of binding to a vector for gene introduction, wherein the method comprises
a step of binding the substance capable of binding to a vector for gene introduction to the nano-particle through some of the functional groups.

14. A method for producing a gene introduction agent, wherein the method comprises a step of binding a vector for gene introduction to a substance capable of binding to a vector for gene introduction in the carrier for gene introduction obtained by the production method according to claim 13.
